Europäisches Patentamt

(19) European Patent Office

Office européen des brevets

(11) Publication number: **0 020 752**
**B1**

(12) **EUROPEAN PATENT SPECIFICATION**

(45) Date of publication of patent specification: **30.03.83**

(51) Int. Cl.³: **C 07 C  67/00, C 07 C  67/05**

(21) Application number: **80900200.9**

(22) Date of filing: **21.12.79**

(86) International application number:
**PCT/US79/01117**

(87) International publication number: ·
**WO 80/01380 10.07.80 Gazette 80/15**

(54) PROCESS FOR PREPARING ALKANOIC ESTERS.

(30) Priority: **26.12.78 US  972855**
**26.12.78 US  972856**
**26.12.78 US  973498**

(43) Date of publication of application:
**07.01.81 Bulletin 81/1**

(45) Publication of the grant of the patent:
**30.03.83 Bulletin 83/13**

(84) Designated Contracting States:
**DE FR NL**

(56) References cited:
**US - A - 3 479 395**
**US - A - 3 637 515**
**US - A - 3 772 383**
**US - A - 3 778 468**
**US - A - 4 016 200**
**US - A - 4 045 373**
**US - A - 4 180 676**

(73) Proprietor: **NATIONAL DISTILLERS AND CHEMICAL CORPORATION**
**99 Park Avenue**
**New York, NY 10016 (US)**

(72) Inventor: **MURIB, Jawad Hamoodi**
**7337 Parkdale Avenue**
**Cincinatti, OH 45237 (US)**

(74) Representative: **Patentanwälte Dipl.-Ing. A. Grünecker, Dr.-Ing. H. Kinkeldey, Dr.-Ing. W. Stockmair, et al,**
**Dr. rer. nat. K. Schumann, Dipl.-Ing. P. Jakob, Dr. rer. nat. G. Bezold Maximilianstrasse 43**
**D-8000 München 22 (DE)**

Courier Press, Leamington Spa, England.

## Process for preparing alkanoic esters

This invention relates to the preparation of alkanoate esters by the catalyzed oxyacylation reaction in the vapor phase of alkanoic acid, oxygen and an olefin, an aromatic hydrocarbon and/or an alkyl substituted aromatic hydrocarbon capable of forming the corresponding alkanoate ester.

The preparation of alkanoate esters by the vapor phase reaction of an alkanoic acid, oxygen and an aromatic or olefinic hydrocarbon in the presence of a noble metal containing catalyst, especially palladium, is well known. Reactions exemplary of the foregoing are shown in U.S. Patent No. 3,190,192 and 3,275,680. Numerous proposals for liquid phase oxyacylation catalysts have been made for use in preparing ethylene glycol diacetate by reacting ethylene, oxygen and acetic acid. By utilizing the catalyst system of the present invention, it is possible to avoid many of the problems associated with the catalyst systems of the prior art in preparing ethylene glycol diacetate and carry out the reaction more desirably in the vapor phase.

The oxyacylation catalysts employed in the process of making alkanoate esters according to this invention overcome in large measure the disadvantages associated with the catalysts of the prior art. Because the catalysts herein can be employed in a vapor phase reaction system and eliminate the presence of noble metals and halogens, there is provided a simpler, more economical and less maintenance-intensive industrial process for efficiently manufacturing a class of organic chemicals of prime economic importance. Moreover, use of the vapor phase facilitates the separation of the resulting ester product from the catalyst.

Ethylene glycol esters are especially a well known class of organic esters having widespread use as solvents and plasticizers. Thus, for example, ethylene glycol diacetate is employed as a solvent for cellulose esters and ethers and is often included in resin, lacquer and printing ink formulations. The monoacetate ester of ethylene glycol is used as a solvent for nitrocellulose. Another principal use for ethylene glycol esters is as intermediates in the preparation of corresponding ethylene glycol, which is one of the most industrially important dihydric alcohols. Other important esters such as allyl acetate and tolylacetate can also be prepared by this invention. In view of the considerable commercial significance of esters, both as useful products in themselves and as intermediates in chemical synthesis, there is an ongoing need to provide processes for preparing the esters in the most economical manner possible.

The present invention relates to the preparation of alkanoate esters by reaction of alkanoic acid, oxygen and an olefin, an aromatic hydrocarbon and/or an alkyl substituted aromatic hydrocarbon capable of forming the corresponding alkanoate ester in the vapor phase at elevated temperatures in the presence of a catalyst system characterized by a catalyst system comprising an oxide of uranium and at least one oxide of arsenic, antimony or bismuth.

The olefins useful in the process of the invention include mono- and diolefins containing from 2 to 8 carbon atoms and may contain impurities of the type and in amounts normally present in the commercial grades thereof. The olefins may contain aromatic, alicyclic or heterocyclic groups and the diolefins may be conjugated or non-conjugated. Among the preferred olefins are those containing from 2 to about 8 carbon atoms, e.g., ethylene, propylene, butene-1, isobutylene, pentene-1, 3-methylbutene-1, hexene-1, butadiene, and the like. Olefins containing up to 6 carbon atoms are particularly preferred.

The aromatic hydrocarbons may be benzene or an alkaryl or aralkyl aromatic hydrocarbon, for example, toluene, xylene, cumene, mesitylene, ethyl benzene and the like. The alkyl group of the aromatic hydrocarbon will generally have 1 to 4 carbon atoms, and there may be more than one such alkyl group on the aromatic hydrocarbon.

Although alkanoic acid employed herein is usually an aliphatic monocarboxylic acid containing about 2 to 8 carbon atoms, preferably 2 to 6, acids having two or more dicarboxylic groups may be used. The alkanoic acids which are especially useful are acetic, propionic, butyric and isobutyric with acetic acid being preferred. It is preferred that the acids contain no more than about 25 weight percent water and preferably less than about 5 weight percent water. Thus, commercial glacial acetic acid which contains less than 2 weight percent water is well suited for use in the reaction. For example, in the preparation of ethylene glycol diacetate the use of large amounts of water admixed with acetic acid may result in hydrolysis of the glycol acetate in the reactor which could complicate the separation procedure.

The oxygen may be pure oxygen gas or alternatively, an oxygen-containing gas mixture such as air or air enriched with oxygen. In addition to these materials the oxygen may contain other inert diluents such as carbon dioxide, nitrogen and the like.

The catalyst system comprises an oxide of uranium and at least one oxide of arsenic, antimony or bismuth. The aforestated oxides can exist in any of their oxidation states and specifically include uranium dioxide, uranyl uranate, uranium trioxide, the trioxides, tetroxides and pentoxides of arsenic, antimony and bismuth, and mixtures of these oxides. The atomic ratio of uranium to antimony, bismuth and/or arsenic can vary over wide limits and advantageously is within the ratio of about 50:1 to about 1:99. A ratio of 10:1 to 1:10 is preferred. U.S. Patent No. 3,198,750 discloses a suitable catalyst containing mixed antimony oxide and uranium oxide and a method for preparing the catalyst.

While it is not necessary to provide the catalyst of this invention with a support, it is generally advantageous to deposit the catalysts upon a carrier such as any of the known and conventional catalyst carrier materials since catalytic efficiency will thereby be significantly improved. Thus, the catalysts herein can advantageously be supported upon silica, alumina, zirconia, silica-alumina, silicon carbide, alundum and inorganic silicate in an amount, by weight of metal of the supported catalyst, of about 1 percent to about 90 percent, preferably about 10 to 50 percent. A preferred catalyst contains about 5% to 30% antimony, bismuth or arsenic, preferably antimony, and 1% to 20% uranium, by weight of the supported catalyst.

The catalyst may be prepared by known techniques, e.g., contacting a metal salt solution with the support drying and calcining in an oxidizing atmosphere, e.g., in the presence of an air sweep. A range of drying temperature of 100°—120°C. and a calcining temperature of 350—900°C. may be suitably employed. Non-supported catalysts may be prepared by precipitation of the soluble salt or salts from solution by, e.g., neutralization with aqueous ammonia solution, filtration, washing, drying and calcining.

A preferred method for preparing a catalyst comprises treating an extruded or pelletized support with an aqueous solution containing uranyl nitrate and/or antimony pentachloride and concentrated hydrochloric acid. The mixture is evaporated and the impregnated support is dried at 110°—120°C. for an additional 12 hours. Examples of suitable water soluble metal compounds which can be used in the preparation of the supported catalysts include uranyl nitrate, uranyl chloride, uranyl sulfate, uranyl tetrachloride, arsenic acid, antimony pentachloride and bismuth nitrate. The catalyst bed can be a fixed bed employing a large particulate or pelleted catalyst or, a fluidized bed.

The catalyst may also contain promoters, such as oxides of molybdenum, thallium, bismuth, copper and silver which are useful, e.g., to improve the selectivity and reaction rate. The amount of promoter, by weight of metal on the supported catalyst, is generally within the range of about 0.1—10%, preferably about 0.5%—3.5%, e.g., 3%.

In general, any apparatus of the type suitable for carrying out reactions in the vapor phase can be used in carrying out the oxyacylation reaction of this invention. The reactor can be brought to the reaction temperature before or after the introduction of the reaction feed mixture. However, in a large-scale operation, it is preferred to carry out the process in a continuous manner and in such a system, the recirculation of unreacted reactants is contemplated.

The concentrations of hydrocarbon, carboxylic acid and oxygen used in the oxyacylation reaction can vary widely. The effective minimum concentration of catalyst will depend upon temperature, residence time and the particular composition of the catalyst employed. The mole ratios of oxygen to hydrocarbon to carboxylic acid fed to the reaction zone is not critical but it should be adjusted so that the mixture used is not in the explosive region. The stoichiometric ratio per ester group formed of hydrocarbon: carboxylic acid: oxygen is 2:2:1 and the relative proportions may vary widely. In general, the hydrocarbon may be used in large excess to improve the selectivity to the ester. Broadly stated, in volume percent, based on the total content of hydrocarbon, carboxylic acid and oxygen, the oxygen is about 1%—50%, the carboxylic acid is about 1%—77% and the hydrocarbon is about 1%—98%. A preferred range is about 1% to 20% oxygen, 2% to 35% carboxylic acid and 45% to 97% hydrocarbon. The feed may also be diluted with nitrogen or other inert gas to maintain the mixture outside the explosive limits.

Although the large amounts of water in the reactor could present problems in some cases, frequently, it is desired for water to be present in the reaction and may be present in amounts up to about 25% by volume. The water may be added to the reactor as a separate recycle stream or vaporized recovered unreacted carboxylic acid.

In general, the temperature in the reaction zone is about 150—450°C., preferably about 250°—400°C, although higher or lower temperatures may be employed.

A wide range of pressures can be adopted. Pressures up to 71.9 Bars or higher, may be employed but will not normally be used because of equipment limitation and increased cost. When an olefin is a reactant the pressure in the reaction zone should be above about 3,8 Bars preferably about 4,2 Bars. The pressure can preferably range from 4,2 to 15,5 Bars with the more preferred range being from 7,0 to 8,8 Bars. When an aromatic hydrocarbon is the reactant the preferred range is from atmospheric to 15,5 Bars, with the more preferred range being atmospheric to 6,7 Bars.

The reaction time will depend largely upon the concentration of reactants and, therefore, may suitably vary over a wide range. Flow rates are preferably adjusted so that the contact time is in the range of about 0.1 to about 60 seconds, and preferably between 1 and 5 seconds. The product from the reaction may be recovered by known methods.

The following expressions as used herein are defined as follows:

The term "% Conversions" means

$$\frac{\text{Millimoles (MM) in} - \text{MM out}}{\text{MM in}} \times 100$$

of the specified reactant.

The term "% Selectivity Component" means

$$\frac{\text{MM Component}}{\text{MM specified material reacted}} \times 100$$

and the term "Catalyst Utility" means grams component produced/liter catalyst/hour (g/l. cat/hr).

Example 1
Preparation of catalyst

Extruded silica gel, 429,5 grams (g.) (3,2 mm diameter×6,4 mm long, 200 m²/g surface area) was placed in a 2-litre beaker and treated with an aqueous solution containing 284,8 g. SbCl$_5$, 90.6 g. UO$_2$(NO$_3$)$_2$ . 6H$_2$O and 210 g. concentrated HCl. While mixing, the mixture was heated to dryness on a hot plate. The resulting impregnated extrudates were calcined in air at 450°C. for 12 hours followed by calcining in air at 850°C for an additional 12 hours.

Oxyacylation of ethylene with acetic acid and oxygen at 8,8 bars and 290°C

15.1 g. of the catalyst prepared in Example 1, was packed in a stainless steel reactor (2.5 cm×12 cm) provided with a thermocouple imbedded in the catalyst bed. The catalyst was heated at 290°C. A gaseous mixture consisting, by volume, of 72.4% ethylene, 25.1% glacial acetic acid and 2.5% oxygen was passed through the heated catalyst at a pressure of 8,8 Bars and a contact time of three seconds. The reactor effluent was passed through three traps connected in series; one cooled with ice water and two traps held at dry-ice temperature. The liquid condensates were combined and analyzed for esters and free acetic acid (by alkaline titration and gas chromatography). The vent gases were analyzed by gas chromatography for ethylene, oxygen and carbon oxides. The analyses showed that 1,2-diacetoxyethane (ethylene glycol diacetate) was produced at the rate of 116 g/l. cat/hr in 62.8% Selectivity based on reacted acetic acid with complete conversion of oxygen (limiting reactant). A small amount of gamma-butyrolactone (1 g/l. cat/hr) was detected in the liquid condensate.

Example 2
Oxacylation of ethylene with acetic acid and oxygen at 7.0 Bars and 300°C

Example 1 was repeated except that the reaction was carried out at 7.0 Bars and 300°C. The production rate of 1,2-diacetoxyethane was 113 g/l. cat/hr at 44.9% selectivity based on reacted acetic acid, with complete conversion of oxygen.

Example 3
Oxyacylation of ethylene with acetic acid and oxygen at 4.2 Bars and 300°C

Example 2 was repeated except that the reactor pressure was 4,2 Bars and the contact time was increased to four seconds. The production rate of 1,2-diacetoxyethane was 24.7 g/l. cat/hr at 69.4% selectivity based on reacted acetic acid. The oxygen conversion was 66.5%.

Example 4
Oxyacetylation of propylene with acetic acid and oxygen at 8,8 Bars and 290°C

15.1 g of the catalyst prepared in Example 1 was packed in a stainless steel reactor (2.5 cm×12 cm) provided with a thermocouple imbedded in the catalyst bed. The catalyst was heated at 290°C. A gaseous mixture consisting of, by volume, 72.4% propylene, 25.1% glacial acetic acid and 2.5% oxygen was passed through the heated catalyst at a pressure of 8,8 Bars and a contact time of three seconds. The reactor effluent was passed through three traps connected in series: one cooled with ice water and two traps held at dry-ice temperature. The liquid condensates were combined and analyzed for esters and free acetic acid (by alkaline titration and gas chromatography). The vent gases were analyzed by gas chromatography for propylene, oxygen and carbon oxides. The analyses showed that allyl acetate was produced along with small amounts of 1,2-diacetoxypropane, $\gamma$-valerolactone and isopropyl acetate.

Example 5
Preparation of catalyst

Extruded silica gel, 429.5 gram (g.) 3,2 mm diameter×6,4 mm long, 200 m²/g surface area) was placed in a 2-liter beaker and treated with an aqueous solution containing 248.8 grams (g.) SbCl$_5$, 90.6 g. UO$_2$ (NO$_3$)$_2$ · 6H$_2$O and 210 g. concentrated HCl. While mixing, the mixture was heated to dryness on a hot plate. The resulting impregnated extrudates were calcined in air at 450°C for twelve hours followed by calcining in air at 850°C for an additional twelve hours.

Oxyacetylation of benzene with acetic acid and oxygen at 1,1 Bars and 350°C

A 30 milliliter (ml.) stainless steel reactor 10 centimeter (cm)×2 cm. (inside diameter) with a thermocouple was packed with 14.3 g. of the catalyst prepared above. A gaseous stream of 629 cubic centimeters (cc.)/minute of, by volume, 10% benzene, 10% acetic acid, 5% oxygen and 75% nitrogen was passed through the reactor and contacted with the catalyst at one atmosphere pressure and about 350°C. The reactor effluent was bubbled through a U-tube held at 0°C. The noncondensable gases were measured by a wet-test meter and a sample of the gaseous mixture was analyzed for oxygen and carbon oxides (CO and CO$_2$). Analysis of the condensate disclosed formation of phenyl acetate as established by gas-liquid chromatography and mass spectroscopy.

## Claims

1. A process for preparing alkanoate esters by reacting alkanoic acid, oxygen and an olefin, an aromatic hydrocarbon and/or an alkyl substituted aromatic hydrocarbon capable of forming the corresponding alkanoate ester in the vapor phase at elevated temperatures in the presence of a catalyst system characterised by a catalyst system comprising an oxide of uranium and at least one oxide of arsenic antimony or bismuth.

2. A process according to claim 1 wherein the catalyst is an oxide of uranium and an oxide of antimony.

3. A process according to claim 2 wherein the catalyst contains 5—30% antimony and 1 to 20% uranium by weight of the total metal.

4. A process according to any of claims 1—3, wherein the catalyst contains a promoter.

5. A process according to any of claims 1—4, wherein the catalyst is supported.

6. A process according to any of claims 1—5, wherein the hydrocarbon is an olefin containing up to 6 carbon atoms.

7. A process according to claim 6 wherein the olefin is ethylene or propylene.

8. A process according to claim 6 or 7 wherein the pressure is above 3,8 Bars.

9. A process according to claim 8 wherein the pressure is from 4,2 to 15,5 Bars.

10. A process according to any of claims 1—5 wherein the hydrocarbon is benzene or an aromatic alkyl substituted hydrocarbon, the alkyl group containing up to 4 carbon atoms.

11. A process according to claim 10 wherein the aromatic alkyl substituted hydrocarbon is toluene.

12. A process according to claim 10 or 11 wherein the pressure is from atmospheric to 15,5 Bars.

13. A process according to any of claims 1—12 wherein the temperature is from 150 to 450°C.

14. A process according to any of claims 1—13 wherein the alkanoic acid is acetic acid.

15. A process according to any of claims 1—14 wherein the volume percent of the oxygen is 1 to 50%, the alkanoic acid is 1 to 77% and the hydrocarbon is 1 to 98%.

## Revendications

1. Procédé de préparation d'esters alcanoïques par réaction d'un acide alcanoïque, d'oxygène et d'une oléfine, d'un hydrocarbure aromatique et/ou d'un hydrocarbure aromatique à substituant alkyle capable de former l'ester alcanoïque correspondant, en phase vapeur, à température élevée, en présence d'un système catalytique, caractérisé par un système catalytique comprenant un oxyde d'uranium et au moins un oxyde d'arsenic, d'antimoine ou de bismuth.

2. Procédé selon la revendication 1, dans lequel le catalyseur est formé d'un oxyde d'uranium et d'un oxyde d'antimoine.

3. Procédé selon la revendication 2, dans lequel le catalyseur contient 5 à 30% d'antimoine et 1 à 20% d'uranium, par en poids de métal total.

4. Procédé selon l'une quelconque des revendications 1 à 3, dans lequel le catalyseur contient un promoteur.

5. Procédé selon l'une quelconque des revendications 1 à 4, dans lequel le catalyseur est supporté.

6. Procédé selon l'une quelconque des revendications 1 à 5, dans lequel l'hydrocarbure est une oléfine contenant jusqu'à 6 atomes de carbone.

7. Procédé selon la revendication 6, dans lequel l'oléfine est l'éthylène ou le propylène.

8. Procédé selon l'une quelconque des revendication 6 et 7, dans lequel la pression est supérieure à 3,8 bar.

9. Procédé selon la revendication 8, dans lequel la pression est de 4,2 à 15,5 bar.

10. Procédé selon l'une quelconque des revendication 1 à 5, dans lequel l'hydrocarbure est le benzène ou un hydrocarbure aromatique à substituant alkyle, le groupe alkyle contenant jusqu'à 4 atomes de carbone.

11. Procédé selon la revendication 10, dans lequel l'hydrocarbure aromatique à substituant alkyle est le toluène.

12. Procédé selon l'une quelconque des revendications 10 et 11, dans lequel la pression est comprise entre la pression atmosphérique et 15,5 bar.

13. Procédé selon l'une quelconque des revendications 1 à 12, dans lequel la température est de 150 à 450°C.

14. Procédé selon l'une quelconque des revendications 1 à 13, dans lequel l'acide alcanoïque est l'acide acétique.

15. Procédé selon l'une quelconque des revendications 1 à 14, dans lequel le pourcentage d'oxygène en volume est de 1 à 50%, celui d'acide alcanoïque de 1 à 77% et celui d'hydrocarbure de 1 à 98%.

## Patentansprüche

1. Verfahren zur Herstellung von Alkanoatestern durch Umsetzung von Alkansäure, Sauerstoff und einem Olefin, einem aromatischen Kohlenwasserstoff und/oder einem alkylsubstituierten aromatischen Kohlenwasserstoff, der den entsprechenden Alkanoatester bilden kann, in der Dampfphase bei erhöhten Temperaturen in Gegenwart eines Katalysatorsystems, gekennzeichnet durch ein Katalysatorsystem, das ein Uranoxid und wenigstens ein Oxid von Arsen, Antimon oder Wismuth enthält.

2. Verfahren nach Anspruch 1, wobei der Katalysator ein Uranoxid und ein Antimonoxid ist.

3. Verfahren nach Anspruch 2, wobei der Katalysator 5 bis 30% Antimon und 1 bis 20% Uran, bezogen auf das Gewicht des gesamten Metalles, enthält.

4. Verfahren nach einem der Ansprüche 1 bis 3, wobei der Katalysator einen Promotor enthält.

5. Verfahren nach einem der Ansprüche 1 bis 4, wobei der Katalysator einen Träger enthält.

6. Verfahren nach einem der Ansprüche 1 bis 5, wobei der Kohlenwasserstoff ein Olefin mit bis zu 6 Kohlenstoffatomen ist.

7. Verfahren nach Anspruch 6, wobei das Olefin Äthylen oder Propylen ist.

8. Verfahren nach Anspruch 6 oder 7, wobei der Druck über 3,8 bar liegt.

9. Verfahren nach Anspruch 8, wobei der Druck 4,2 bis 15,5 bar beträgt.

10. Verfahren nach einem der Ansprüche 1 bis 5, wobei der Kohlenwasserstoff Benzol oder ein alkylsubstituierter aromatischer Kohlenwasserstoff ist, wobei die Alkylgruppe bis zu 4 Kohlenstoffatome enthält.

11. Verfahren nach Anspruch 10, wobei der alkylsubstituierte aromatische Kohlenwasserstoff Toluol ist.

12. Verfahren nach Anspruch 10 oder 11, wobei der Druck von Atmosphärendruck bis 15,5 bar ist.

13. Verfahren nach einem der Ansprüche 1 bis 12, wobei die Temperatur 150 bis 450°C beträgt.

14. Verfahren nach einem der Ansprüche 1 bis 13, wobei die Alkansäure Essigsäure ist.

15. Verfahren nach einem der Ansprüche 1 bis 14, wobei der Volumenprozentsatz des Sauerstoffs 1 bis 50%, der Alkylsäure 1 bis 77% und des Kohlenwasserstoffes 1 bis 98% beträgt.